Europäisches Patentamt

(19) European Patent Office

Office européen des brevets

(11) Publication number: **0 129 669**
A2

(12) # EUROPEAN PATENT APPLICATION

(21) Application number: **84104721.0**

(22) Date of filing: **26.04.84**

(51) Int. Cl.⁴: **G 01 N 33/74**

(30) Priority: **27.04.83 US 488990**

(43) Date of publication of application:
**02.01.85 Bulletin 85/1**

(84) Designated Contracting States:
**DE FR GB NL SE**

(71) Applicant: **THE WELLCOME FOUNDATION LIMITED**
**183-193 Euston Road**
**London NW1 2BP(GB)**

(72) Inventor: **Parikh, Indu**
**2448 Booker Creek Road**
**Chapel Hill North Carolina 27514(US)**

(72) Inventor: **Moncharmont, Bruno**
**16 Rue Pasteur**
**F-75011 Paris(FR)**

(74) Representative: **Berg, Wilhelm, Dr. et al,**
**Patentanwälte Dr. Berg Dipl.-Ing. Stapf Dipl.-Ing.**
**Schwabe Dr. Dr. Sandmair Postfach 86 02 45**
**Stuntzstrasse 16**
**D-8000 München 86(DE)**

(54) An estrogen receptor assay and test kit therefor.

(57) A process and kit for the determination of the amount of estrogen receptors in a tissue sample by separating the nuclei from the tissue sample after preferably causing at least some of the estrogen receptors not already associated with the nuclei to become associated therewith and measuring the amount of labeled antibodies bound to the estrogen receptor associated with the nuclei. The invention is useful in determining the proper treatment in cases involving hormonal therapy which is often appropriate with patients having mammary cancer.

EP 0 129 669 A2

A Novel Estrogen Receptor Assay

**TITLE MODIFIED**
**see front page**

Surgery followed by a program of chemotherapy is a widely practiced regimen for breast cancer. When the neoplasm is detected early the extent of surgery can be minimized and chemotherapy can halt the future growth and spreading of cancer cells in many cases. The physician's choice of chemotherapy agents is based on several factors. One such factor is the level of estrogen receptors ($E_2R$) in the malignant cells. When the $E_2R$ level is high, hormonal therapy can be used as such or as an adjuvant to treatment with antineoplastic agents, but if the $E_2R$ level is low, hormonal therapy is ineffective.

Estrogen receptors have recently been identified in carcinomas of the intestine and of the skin. Although these are not typical target tissues for estrogen, a possible role of the hormone in the neoplastic growth may be postulated. In such case, the measurement of the estrogen receptor levels in the neoplastic tissues would be of value in order to direct post-surgery therapy, in much the same manner as in the well-studied situation with breast cancer.

If the physician has knowledge that the $E_2R$ level is low, the patient can be spared the expense, the delay in initiation of more effective treatment, and side effects of hormonal therapy. In recent years, useful $E_2R$ assays have been developed and are available in the larger hospitals and medical centers. These assays are complex, time consuming and hence relatively expensive and sometimes unreliable. In addition, since these assays measure $E_2R$ levels in the cytosolic fraction only, knowledge of nuclear estrogen receptor levels will complement these measurements to provide a more complete assessment of estrogen receptors.

IP/mm/CFT1/3/1

2

Estrogen receptors have been studied and identified using their steroidal hormone specific binding activity (see Jensen, E. V, and Jacobson, H. I., Recent Prog. Horm. Res. 18, 387-414 (1962) and Toft, D., and Gorski, J., Proc. Natl. Acad. Sci. U.S.A. 55, 1574-1581 (1966). Although the radiolabeled hormone, e.g. estradiol, allows a highly specific and functional identification of the receptor, the binding activity is labile in certain experimental conditions (e.g., heat, detergents, etc.). Furthermore, in some instances the steroid binding site of the receptor is occupied by non-radiolabeled hormone of endogenous or pharmacological origin. In order to circumvent this problem, many assays have been developed that use high temperature (Anderson, J., Clark, J. H. and Peck, Jr., B. J., Biochem. J. 126, 516-567 (1972) or chaotropic salts, Sica, V., Puca, G. A., Molinari, A. M., Buonaguro, F. M., and Bresciani, F., Biochemistry 19, 83-88 (1980)) to cause the exchange of the receptor-bound endogenous hormone with the radiolabeled one. In the recent years, monoclonal antibodies to the estrogen receptor have become available (Moncharmont, B., Su, J. L. and Parikh, I., Biochemistry 21, 6916-6921 (1982). Their interaction with the receptor is independent of the occupation of the estradiol binding site. Also see U.S. Patent 4232001 and Proc. Nat. Acad. Sci. U.S.A. 77, 5115-5119 (1980).

In addition, the method of the present invention also is more sensitive and more accurate than other methods of the art because the assay method of the present invention works at low temperatures and does not require the use of chaotropic salts while providing a means of measuring $E_2R$ levels in the cell nucleus.

The present invention provides a method of determining the estrogen receptor content of the nuclear fraction of estrogen target tissues. For example, a portion of neoplastic tissue taken from a breast, previously diagnosed as affected by cancer and removed by surgery, is homogenized in an appropriate aqueous buffer.

The homogenate suspension is centrifuged to separate a liquid phase and a solid pellet. The pellet is washed and the nuclei isolated by methods known in the art. The isolated nuclei in buffer solution and labeled estrogen specific monoclonal antibodies are incubated to form a complex of the estrogen receptor associated with the nuclei and the labeled antibody. The suspension is then centrifuged to yield a solid pellet and a liquid supernatant. The pellet is washed with buffer and the amount of labeled material is assayed by standard test and the quantity present is determined by methods well known in the art.

The present invention is also useful in measuring estrogen receptor molecules associated with the nuclei after in vitro translocation. For example, neoplastic breast tissue is homogenized in an appropriate aqueous buffer and incubated with $10^{-7}$ to $10^{-9}$ molar estradiol. The estrogen receptor-estradiol complex is then translocated to the nuclei using conditions known in the art. The nuclei are isolated, and incubated with labeled estrogen receptor specific antibodies. This suspension is separated by centrifugation into a solid pellet which contained inter alia, the total receptors complexed with estradiol and the labeled antibody. The $E_2R$ can be measured directly by determining the amount of labeled antibody in the pellet.

The present invention also provides a radioimmunoassay (RIA) or other type of assay using labeled antibodies of the displacement type for the deter-

IP/mm/CFT1/3/3

mination of the $E_2R$ content of a tissue sample. Nuclei having high specific $E_2R$ content are prepared, e.g., from the uteri of cows which have been treated with estradiol. In vitro translocation may be used to increase the $E_2R$ content of the nuclei. Nuclei containing high levels of $E_2R$ may also be obtained from human breast cancer cell lines, e.g., MCF-7, using standard tissue culture techniques by treatment with estradiol and harvesting the cells at an appropriate time.

A standard curve is constructed by incubating labeled estrogen specific monoclonal antibodies, with the nuclei containing $E_2R$ in the presence of serial dilutions of a soluble preparation of known content of soluble estrogen receptor (which may be obtained from the cytosolic fraction prepared from any mammalian estrogen target tissue, as well as from human breast cancer cell line and quantitated using radiolabeled estradiol as is well known in the art).

The assay is carried out by incubating the same amount of the nuclei containing $E_2R$ as used in preparing the standard curve, an appropriate known amount of a solubilized preparation of a target tissue and the same amount of labeled estrogen specific monoclonal antibodies as used in preparing the standard curve. The antibodies competitively bind with the nuclear $E_2R$ forming an insoluble complex and with the $E_2R$ of the solubilized tissue to form a soluble complex. The mixture is centrifuged after a suitable incubation period to pellet the insoluble complex. The radioactivity of the pellet is measured and compared to the standard curve to determine the $E_2R$ content of the target tissue.

## EXAMPLE I  ANTIBODY PREPARATION

### A)  PURIFICATION OF ESTROGEN RECEPTOR

The estradiol receptor complex was purified from calf uteri according to the following procedure.  The uteri were removed from the animals in the slaughterhouse, rapidly cleaned from connective tissue and ovaries, packed in dry ice for the transportation and stored at -120°C until they were processed.  About 300 g of tissue were processed at a time.  The uteri were thawed, ground through a commerical meat grinder and homogenized with 2.5 volumes of buffer A (20 mM Tris·HCl, pH 7.4 containing 2 mM, EDTA and 1 mM, DTT) with various bursts of Polytron homogenizer at 0-4°C until no more pieces of intact tissue were visible.  The homogenate was then centrifuged at 150,000 g for 1 hr and the supernatant (cytosol) was collected.  Sufficient $MgCl_2$ to give a final concentration of 5 mM was added to the cytosol.  Heparin covalently bound to agarose (Heparin-Sepharose; prepared according to the published procedure, see Molinari, A. M. et al., Proc. Natl. Acad. Sci. USA, 74, 4886-4890, 1977) was equilibrated in buffer B (20 mM Tris·HCl, pH 7.4, containing 4 mM $MgCl_2$ and 1 mM DTT ).  Heparin-Sepharose (120 g wet weight) was incubated batchwise with the cytosol for a 2 hr at 4°C in continuious shaking.  The resin was then collected on a Buchner funnel, washed with about 2 liters of buffer B, packed on a wide column (approximately 5 cm diameter) and washed with the same buffer until the optical density at 280 nm in the wash was reduced to 0.005.  The estradiol receptor was eluted with buffer B containing 4 mg/ml of Heparin.  The peak of estradiol binding activity was collected, brought to 100 mM KCl and applied to a 3 ml column of Estradiol-Sepharose equilibrated in buffer A + 0.1M KCl with a flow rate of about 10-20 ml/hr.  Estradiol-Sepharose was prepared by coupling diaminodipropylamine (DADPA) to

cyanogenbromide activated Sepharose 4BCl (Published procedure, Sica, V. et al., J. Biol. Chem. 248, 6543-6558, 1973). The Sepharose-DADPA was then coupled with 17β-estradiol 17-hemisuccinate in presence of carbodiimide. The estradiol-Sepharose column (containing approximately 2 ml resin), loaded with receptor, was carefully washed with 100 ml of buffer A, 50 ml of buffer A + 2M KCl and again 50 ml of buffer A. This washing cycle was repeated twice. Elution of the receptor was performed with 5 ml of buffer A containing 0.5M NaSCN, 5 x $10^{-7}$M [6,7 $^3$H] 17β-estradiol and 10% dimethylformamide. The peak of macromolecular bound estradiol was collected, dialysed against buffer A and concentrated. The yield was 130 μg of receptor with about 20% purity, as tested by polyacrylamide gel electrophoresis.

B)    PREPARATION OF ANTIBODIES

Three Balb/c mice (3 weeks old) were injected subcutaneously with 10 μg of purified receptor emulsified with an equal volume of Freund's complete adjuvant. An equal dose in incomplete Freund's adjuvant was injected twice there after at two-week intervals and again after an additional one-month interval. Ten days after the last injection, each mouse received a daily i.v. injection of the estrogen receptor in the tail for three days. On the fourth day, one mouse was sacrificed and the spleen removed under sterile conditions. A cell suspension in 5 ml HBSS medium was made by passage through a 20-gauge needle for 5-6 times. Red blood cells were lysed with 3 volumes of 0.15M NH4Cl containing 0.01M KHCO3, pH 7.2 at room temperature for 5 min.

Spleen cells (approximately $10^8$) were mixed with 5 x $10^7$ myeloma cell SP2/0/Ag 14 and centrifuged at 1900 rpm for 5 min. The cells were resuspended in 50% polyethyleneglycol (PEG-1000) and diluted to a final 5% concentration of PEG over an 8 min period at 37°C with serum free Dulbecco's MEM medium. The cells were centrifuged again, resuspended in DMEM + 20% fetal calf serum (FCS) at a final concentration of 2.5 x $10^6$ cells/ml. The cells were plated in 250 wells of 96-well microtiter dishes. The next day the medium was changed to hypoxanthine-aminopterin-thymidine (HAT) containing medium for hybrid selection. The cells were refed every other day with HAT medium for 10 days. The medium was then changed to DMEM + 20% FCS; 180 wells were found to contain hybrids. After a further two days, the medium from each well was assayed for antibody to estrogen receptor. The presence of antibody was tested by incubation of the well supernatant with purified estrogen receptor immobilized on 96-well plate which were previously coated with heparin. The presence of antibodies attached to the receptor-heparin complex, after washing the well, was detected by reaction with goat antimouse IgG coupled to a β galactosidase according to the instructions provided by the manufacturer (Bethesda Research Laboratories) of the enzyme labeled goat IgG. The presence of antibody reacting with estrogen receptor in these wells was further confirmed by sucrose gradient analysis.

Cells from some of the positive wells were cloned in soft agar. Visible clones were collected and transferred to 96-well plates. The supernatant of these clones were screened with a double antibody immunoprecipitation assay using [$^{125}$I]-estradiol labeled cytosolic receptor.

Cells of the positive clones were injected i.p. in Balb/c mice ($10^7$ cells/mouse) primed with pristane (3,6,10,14-Tetramethylpentadecane). After 7-10 days, the mice were sacrificed and the ascites fluid collected from the peritoneal cavity. Antibodies were purified by ammonium sulphate precipitation and DEAE cellulose chromatography.

An alternative method in serum-free medium allows the production of small amounts of antibodies of high purity. This is possible because the parental myeloma cell line Sp2/0-Ag14 used for the fusion does not synthesize immunoglobulins (see: Shulman et al., Nature 276, 269-270, 1978). The hybridoma cells were initially grown in DMEM containing 20% FCS up to a concentration of 0.5-1 x $10^6$ cells/ml and then transferred to serum-free medium. After 16-18 hr, the medium was collected and chromatographed on a Protein A-Sepharose column. After extensive washing with PBS, the absorbed immunoglobulins were eluted with 0.1 N acetic acid and immediately neutralized with Tris. Although the purity of antibodies prepared in such fashion is more than 95%, the amount of antibodies produced is less than that produced in ascites.

Analysis of these preparations on SDS-PAGE showed presence of IgG and absence of IgM. The amount of purified IgG obtained from a single mouse varies between 1.5 mg to 20 mg.


EXAMPLE II   RADIOIODINATION OF THE ANTIBODIES

An aliquot of purified monoclonal antibodies was radiolabeled by the lactoperoxidase method (Marchalonis, J. J., Biochem. J., 113, 299-305, 1969). The reaction mixture (total volume 150 µl) contained 30 µg of antibody, 2 mCi Na[$^{125}$I], 50 µg of lactoperoxidase and 0.002% $H_2O_2$ in 0.3 M sodium acetate, pH 5.6. The reaction was started by addition of the hydrogen peroxide and stopped after 2 min at room temperature by addition of 100 µl of tyramine hydrochloride (2 mg/ml), followed after 1 min by 100 µl of NaI (2 mg/ml). The radiolabeled antibody was purified on a Sephadex G-100 column (10 ml) equilibrated in 50 mM phosphate buffer, pH 7.5, containing bovine serum albumin (4 mg/ml) and the fractions in the void volume were pooled. The specific activity of the radiolabeled antibody was in range of 10-50 µCi/µg. Similarly, the antibody may be labeled with other radionucleides, such as $^3H$ or $^{14}C$. It is also possible to produce antibodies labeled with [$^{35}S$]methionine. For this purpose the hybridoma cells were resuspended in methionine-free medium containing 50 µCi of

[$^{35}S$]methionine (600-1400 Ci/mmol) per ml of cell culture medium. The $^{35}S$-labeled antibodies were purified by Protein A-Sepharose column as described above.


EXAMPLE III    PREPARATION OF ANTIBODIES WITH NON-ISOTOPIC LABELS

Instead of a radiolabel, the antibody can also be labeled with an enzyme, a fluorophore or a chromophore by methods known in art for labeling proteins. Examples of coupling of biological substances to enzymes are described in, for example, Steinberger, L. A., _Immunocytochemistry_, Prentice Hall, New York (1974).

A)    PREPARATION OF HORSERADISH PEROXIDASE-ANTIBODY COMPLEX

Five milligrams horseradish peroxidase enzyme are dissolved in 1 ml of 0.3 M $NaHCO_3$, pH 8.1 and 0.1 ml of 1% solution of fluorodinitrobenzene in absolute methanol is added. After incubation for 1 hour at room temperature, 1.0 ml of 0.5 M $NaIO_4$ in water is added and the mixture stirred at room temperature for 30 min.  Ethylene glycol (1.0 ml, 0.15 M in distilled water) is added and after 1 hour stirring at room temperature, the solution dialyzed against 10 mM $Na_2CO_3$, pH 9.5 at 4°C.  To the dialyzed solution 5 mg antibody is added and the mixture further stirred at room temperature followed by addition of 5 mg $NaBH_4$.  The mixture is left at 4° for overnight.  The reaction mixture is then dialyzed and chromatographed through Sephadex G-100 column.  The fractions containing the enzyme-antibody complex are pooled (more detailed description of this procedure is given by Nakane, P. K., et al. J. Histochem. Cytochem. 22, 1084-1091 1974.)  Various other enzymes may be coupled to the antibody by methods known in the art.


## B) PREPARATION OF FLUOROPHORE CONJUGATED ANTIBODY

The purified monoclonal antibody (5 mg), dissolved in 2 ml 0.1 M $NaHCO_3$

pH 8.1, was mixed with 15 µl of a solution of Texas Red in dimethylsulfoxide (1 mg/ml) for overnight at 4° in the dark.  The red solution was then chromatographed through a 20 cm x 1 cm Sephadex G-25 column and the void volume peak was collected and used in biochemical assays.  Various other

fluorophores or chromophores may be coupled to the antibody by methods known in the art (see Wang, K., et al., Methods in Enzymology, 85B, 514, 1982).


## EXAMPLE IV    ASSAY FOR $E_2R$ CONTENT IN AN UNKNOWN SAMPLE OF NUCLEI

An explant of mammalian tissue was weighed (for example 500 mg) and homogenized with polytron homogenizer in ice-cold TEDS buffer (20 mM

Tris HCl, pH 7.4, containing 1 mM EDTA, 1 mM dithiothreitol and 10% [w/v] sucrose).  The homogenate was filtered through a nylon mesh (200 µm) and centrifuged at 800 x g for 20 min. The nuclear pellet was washed twice with the same buffer and resuspended in TEDS buffer at 0.2-0.5 mg DNA/ml.

A 100 μl-aliquot of the nuclear suspension was incubated in duplicate for 3 hr with an appropriate dilution (20 ng per assay) of the radio-iodinated antibody in presence or absence of 5 μg/assay of unlabeled antibody (from the same clone) in a final volume of 200 μl. Antibodies (both unlabeled as well as labeled) were diluted in TEDS buffer containing 1 mg/ml of bovine γ-globulin. At the end of the incubation, 4 ml of ice-cold TEDS buffer containing 1 mg/ml bovine serum albumin was added to each tube, followed by centrifugation at 800 x g for 20 min. The nuclear pellet was washed once with the above mentioned buffer and counted for radioactivity in a gamma counter.

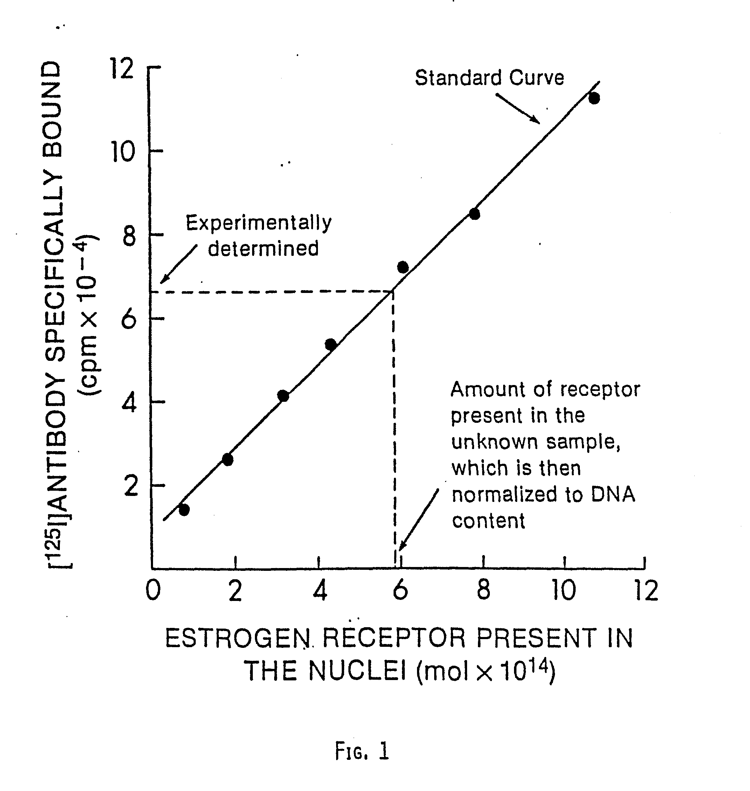

A standard curve was constructed simultaneously in the same conditions using a stabilized preparation of nuclei containing known predetermined amount of estrogen receptor (5 to 100 fmol). The amount of estrogen receptor present in the unknown sample was determined with the use of a standard curve (see Figure 1). Such value of the estrogen receptor content present in the sample has to be normalized to the DNA content. The DNA content of the sample was measured (for details see: Burton, K., Biochem. J. 62, 315-323, 1956) by preincubating a 100 μl aliquot of the nuclear suspension in 0.5 M perchloric acid (final volume 300 μl) for 15 min at 70°C, followed by addition of 1 ml of diphenylamine reagent (1.5 g diphenylamine, 1.5 ml $H_2SO_4$, 100 μl acetic acid and 0.5 ml of 1.6% acetaldehyde) and incubation for 18 hr at 30°C. The optical density value at 600 nm of the sample was then compared with a standard curve obtained with samples of known DNA content.

IP/mm/CFT1/3/11

EXAMPLE V   <u>PREPARATION OF NUCLEI OF ESTROGEN TARGET TISSUE CONTAINING KNOWN</u>
<u>AMOUNT OF ESTROGEN RECEPTOR.</u>

An ovariectomized rat was injected with a dose of 17β-estradiol (e.g., 10-50 μg/kg body weight). After 1 hr the animal was sacrificed and the uterus removed, weighed and homogenized in 10 volumes of TEDS buffer containing 0.1 mM Phenylmethylsulphonylfluoride. The homogenate was filtrated through a nylon mesh (200 μm), centrifuged at 800 x g for 20 min. and the pellet washed twice with the same buffer. The pellet was then resuspended in 2 ml of homogenization buffer and layered onto a 2 ml-cushion of TED buffer containing 2.1 M sucrose and centrifuged in a swinging bucket rotor at 45,000 x g for 60 min. The pellet was washed once and resuspended in 2 ml of TEDS buffer. The amount of estrogen receptor present in a fixed aliquot of such preparation was determined as follows. A 100 μl aliquot was incubated with 15 nM 17β-[$^3$H]estradiol in TEDS buffer containing 0.5 M NaSCN in absence or presence of a 500-fold excess of unlabeled hormone in a final volume of 300 μl. After incubation at 4°C for 15-18 hours, 0.3 ml of ice cold charcoal suspension (1% charcoal, 0.1% dextron T-70 and 0.1% gelatin in TEDS buffer) was added to each tube and, after an additional 15 min free from bound estradiol was separated by centrifugation at 50,000 rpm for 30 min. Radioactivity present in a 0.3 ml aliquot of the supernatant was determined and the specific binding activity computed by subtracting from the binding activity of the samples with radioactive hormone the binding activity of the samples containing the excess of unlabeled hormone. The amount in moles of estrogen receptor present is calculated from the specific activity of the radiolabeled estradiol.

The following abbreviations are used herein:

CNBr: Cyanogen bromide; DADPA: Diaminodipropylamine; DMEM: Dulbecco's minimum essential medium; DTT: Dithiothreitol; $E_2R$: Estrogen Receptor; EDTA: Ethylenediaminotetraacetic acid; HBSS: Hank's balanced salt solution; IgG: Gamma immunoglobulin of G type; IgM: Gamma immunoglobulin of M type; MEM: Minimum essential medium; PAGE: Polyacrylamide gel electrophoresis; PBS; Phosphate buffered saline; SDS: Sodium dodecyl sulphate and TRIS: Tris(hydroxymethyl)aminomethane

*14*

## Brief Description of the Drawing

Fig. 1 represents a standard and prepared in accordance with this invention together with an indication of how the standard curve is used.

Standard curve is produced by incubation of different amount of estrogen receptor present in nuclei with a fixed amount of radiolabeled antibody. The specific binding of the antibody refers to the difference between the total binding and the binding in presence of an excess of unlabeled antibody. The amount of estrogen receptor present in the unknown sample is determined from the abscissa value corresponding to the experimentally determined specific binding of [$^{125}$I] antibody (ordinate value) with the use of the standard curve.

IP/mm/CFT1/3/14

WE CLAIM:

1. A method of measuring the quantity of estrogen receptor associated with the nuclei of target tissue which comprises separating the nuclei from the target tissue and measuring the amount of estrogen receptor associated with ther nuclei by use of a labeled antibody.

2. A method of measuring the amount of estrogen receptors which comprises mixing together nuclei containing a fixed amount of estrogen receptors, an amount of unknown soluble estrogen receptors and labeled antibodies and measuring the binding of the antibodies attached to the estrogen receptors associated with nuclei.

3. A process for the determination of the quantity of estrogen receptors in a tissue sample containing nuclei comprising administering estradiol to a mammal from which a tissue sample is to be taken, taking a tissue sample from the mammal, isolating intact nuclei from the tissue sample, adding labeled estrogen specific antibodies to the separated intact nuclei and then determining the level of binding of labeled antibody to said estrogen receptors.

4. A method of claim 1 in which estradiol is mixed with the target tissue prior to separating the nuclei from the target tissue.

5. A kit for use in determining the levels of estrogen receptors in mammalian tissues comprising a) a quantity of estradiol sufficient to cause translocation of the estrogen receptor to the nuclei of the tissue, b) a quantity of antibody labeled with a detectable tag and c) a stabilized nuclei preparation containing a predetermined quantity of estrogen receptor.

6. A kit for use in determining the levels of estrogen receptors in mammalian tissues comprising a) a quantity of labeled antibody, b) a quantity of nuclei containing a fixed amount of estrogen receptor and c) a soluble estrogen receptor preparation.

IP/mm/CFT1/3/17

7. The kit of claim 5 or 6 in which each of the quantities is stored in a sealed container of plastic or glass.

//1

FIG. 1

IP/mm/CFT1/3/15